(19) Europäisches Patentamt / European Patent Office / Office européen des brevets

(11) **EP 4 273 220 A1**

(12) **EUROPEAN PATENT APPLICATION**
published in accordance with Art. 153(4) EPC

(43) Date of publication:
**08.11.2023 Bulletin 2023/45**

(21) Application number: **21915666.8**

(22) Date of filing: **23.12.2021**

(51) International Patent Classification (IPC):
**C12N 1/16** (2006.01)    **C12P 21/02** (2006.01)

(52) Cooperative Patent Classification (CPC):
**C12N 1/16; C12P 21/02**

(86) International application number:
**PCT/KR2021/019679**

(87) International publication number:
**WO 2022/145868 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.12.2020 KR 20200188450**

(71) Applicant: **Samyang Corporation**
**Jongno-gu**
**Seoul 03129 (KR)**

(72) Inventors:
• **KIM, Jin-Ha**
**Hongseong-gun, Chungcheongnam-do 32272
(KR)**
• **KWON, Soun Gyu**
**Gwangmyeong-si, Gyeonggi-do 14311 (KR)**
• **CHOI, Eunsoo**
**Seongnam-si, Gyeonggi-do 13588 (KR)**
• **PARK, Bu-Soo**
**Hanam-si, Gyeonggi-do 13014 (KR)**
• **AHN, Sin Hye**
**Goyang-si, Gyeonggi-do 10374 (KR)**

(74) Representative: **Wohlfahrt, Jan Günther et al
Gleiss Große Schrell und Partner mbB
Patentanwälte Rechtsanwälte
Leitzstraße 45
70469 Stuttgart (DE)**

(54) **YEAST EXTRACT CONTAINING TRIPEPTIDE AND PREPARATION METHOD THEREFOR**

(57)     The present invention relates to a yeast extract having a high tripeptide content and a high dry matter content using a yeast cell containing tripeptide and a method for preparing the same.

【FIG.3】

EP 4 273 220 A1

**Description**

**[TECHNICAL FIELD]**

The present invention relates to a yeast extract having a high tripeptide content and a high content of dry product using a yeast cell containing tripeptide, and a preparation method thereof.

**[RELATED ART]**

Glutathione (L-γ-glutamyl-L-cysteineylglycine, GSH) is a physiologically active substance present in cells and is in the form of a tripeptide composed of three amino acids of glutamate, cysteine, and glycine. In the body, it exists in two forms: reduced glutathione (GSH) and oxidized glutathione (GSSG). Glutathione exists in the concentration of 0.1 to 10 mM in the cells of animals, plants and microorganisms, and accounts for more than 90% of the whole non-proteinaceous components of cells. The various functions of glutathione are important not only in agriculture but also in many medical fields including enzymology, transport, pharmacology, therapy, toxicology, endocrinology and microbiology.

Glutathione can be produced mainly by fermentation using microorganisms or enzyme synthesis process. Due to the high production cost, the enzyme synthesis process has not yet been commercialized, whereas the method of culturing microorganisms and extracting glutathione from cells is widely used industrially. As microorganisms for glutathione production, yeasts that contain high intracellular glutathione and are recognized as safe microorganisms for food production have been widely used. In particular, Saccharomyces genus strains and Candida genus strains are representative. In the case of wild-type strains of these yeasts, the advantages that the glutathione concentration is already quite high, about 0.1-1 % of dry weight, and highdensity cell culture and rapid growth in low-cost media make fermentative production method using yeast competitive.

**[DETAILED DESCRIPTION OF THE INVENTION]**

**[Technical Problem]**

An embodiment of the present invention relates to a tripeptide extract having a high content of tripeptide, such as glutathione extract, and a method for preparing the same.

A further embodiment of the present invention relates to a yeast extract having a high dry matter content while maintaining the tripeptide content, and a method for preparing the same.

A further embodiment of the present invention is to provide a food, food additive, beverage, beverage additive, health functional food, or pharmaceuticals using a yeast extract having a high tripeptide content and a high dry matter content.

**[Technical Solution]**

An embodiment of the present invention relates to a tripeptide extract obtained from yeast cell containing a high content of tripeptide, such as glutathione extract, and a method for preparing the same.

A further embodiment of the present invention includes a solvent extract obtained by extracting glutathione-containing yeast cells or suspension of the cells using water at a temperature of 60 to 90 °C, and relates to a yeast extract containing glutathione, where the glutathione content is preferably 0.5 to 20 wt% of glutathione based on the solid content.

A further embodiment of the present invention is a method for producing a yeast extract containing glutathione, comprising a step of obtaining a solvent extract obtained by extracting glutathione-containing yeast cells or suspension of the cells using water at a temperature of 60 to 90 ° C. Preferably, the glutathione content is 0.5 to 20 wt% of glutathione based on the solid content.

In another embodiment, the solvent extract may be extracted with water by treating yeast cells with a proteolytic enzyme, and in detail, the primary extract obtained by extracting glutathione-containing yeast cells with hot water at 60 to 90 ° C, and a secondary extract obtained by treating yeast cells recovered from the primary extract with a proteolytic enzyme and then extracting with water.

The present invention relates to glutathione extraction using yeast containing glutathione and glutathione extract prepared therefrom, wherein the yeast extract is prepared in high yield using a hot water extraction method, and optionally hot water extraction and proteolytic enzyme treatment may be performed to obtain an extract with a higher yield, and may be used in various applications such as food and health functional products by preparing a yeast extract having a high protein content.

As yeast extracts are produced with interest in application to nucleic acids and seasoning materials, the present invention intends to provide a method for producing yeast extracts containing tripeptide (glutathione) through effective extraction of yeast cells by heat treatment and enzyme treatment for the yeast cells.

Hereinafter, the present invention will be described in more detail.

An embodiment of the present invention relates to a glutathione-containing yeast extract, comprising a solvent extract obtained by extracting glutathione-containing yeast cells or suspension of the cells with hot water at a temperature of 60 to 90 ° C.

A further example of the present invention relates to a glutathione extract, or a yeast extract containing glutathione, comprising a step of obtaining a solvent extract by extracting glutathione-containing yeast cells or suspension of the cells with hot water at a temperature of 60 to 90 °C.

The yeast extract has a glutathione content of 0.5 to 20 wt%, 1 to 20 wt%, 1.5 to 20 wt%, 2.0 to 20 wt%, 2.5 to 20 wt%, 3.0 to 20 wt%, 3.5 to 20 wt%, 4.0 to 20 wt%, 4.5 to 20 wt%, 0.5 to 18 wt%, 1 to 18 wt%, 1.5 to 18 wt%, 2.0 to 18 wt%, 2.5 to 18 wt%, 3.0 to 18 wt%, 3.5 to 18 wt%, 4.0 to 18 wt%, 0.5 to 16 wt%, 1 to 16 wt%, 1.5 to 16 wt%, 2.0 to 16 wt%, 2.5 to 16 wt%, 3.0 to 16 wt%, 3.5 to 16 wt% , 4.0 to 16 wt%, 4.5 to 16 wt%, 0.5 to 15 wt%, 1 to 15 wt%, 1.5 to 15 wt%, 2.0 to 15 wt%, 2.5 to 15 wt%, 3.0 to 15 wt%, 3.5 to 15 wt% %, 4.0 to 15 wt%, 0.5 to 14 wt%, 1 to 14 wt%, 1.5 to 14 wt%, 2.0 to 14 wt%, 2.5 to 14 wt%, 3.0 to 14 wt%, 3.5 to 14 wt%, 4.0 to 14 wt%, or 4.5 to 14 wt% based on the solid content. In the present invention, the yeast extract with a high glutathione content can be prepared by extracting glutathione with a high content and yield by using yeast containing glutathione in the cell as an extraction raw material because the yeast cell has the ability to produce tripeptide, such as glutathione.

The yeast extract may be a solvent extract itself, a concentrated liquid, or a dried product obtained by additionally performing a drying process. The concentration and drying processes are not particularly limited, but the treatment at a low temperature is preferable due to inclusion of peptides and proteins, and includes, for example, hot air drying or freeze drying.

The extraction raw material for preparing the yeast extract may be yeast containing glutathione, and specifically, may be a yeast cell or a suspension of the cell. The yeast may be at least one selected from the group consisting of strains of the genus Saccharomyces (e.g., *Saccharomyces cerevisiae*), strains of the genus Candida (e.g., *Candida utilis*), and strains of the genus *Pachia.* Preferably, it may be *Candida utilis.*

When using a yeast cell suspension as the extraction raw material, the cell concentration is 50 to 500, 50 to 450, 50 to 400, 50 to 350, 100 to 500, 100 to 450, 100 to 400, 100 to 350, 150 to 500, 150 to 450, 150 to 400, or 150 to 350 of optical density (OD) value measured at 600 nm. Alternatively, the cell concentration may be 40 to 200 g/L, or 40 to 160 g/L based on dry cell weight (DCW). The cell suspension may be prepared by suspending the microbial cells in water, and specifically, may be prepared by recovering the microbial cells from the microbial cell culture medium and suspending the microbial cells in water.

The solvent extract is prepared by extracting with water at 60 to 90 °C. When ethanol is used as an extraction solvent, yeast extracts used as foods or medicines require a step of removing ethanol, so there is a limit to industrial application and a problem of low productivity. In the present invention, the yeast extract is prepared by water as the extraction solvent, and thus can be consumed immediately and the water can be easily removed by simply drying, which is advantageous for industrial applications.

The extraction temperature of the yeast extract can be adjusted using water of 60 to 90 °C, for example, 60 to 90 °C, 62 to 90 °C, 64 to 90 °C, 60 to 98 °C, 62 to 80 °C, 64 to 80 °C, 60 to 75 °C, 62 to 75 °C, 64 to 75 °C, or 65 to 75 °C, but is not limited thereto.

In a further example of the present invention, the step of obtaining a solvent extract of extracting glutathione-containing yeast cells or suspension of the cells using water at a temperature of 60 to 90 ° C can be a product obtained by treating the cells with protease and extracting the cells with water.

Specifically, the solvent extract may include a primary extract obtained by extracting glutathione-containing yeast cells with water at 60 to 90 ° C. and a secondary extract obtained by treating the cells recovered from the primary extract with a proteolytic enzyme to and extracting them with water. More specifically, the solvent extract may be a mixture of a supernatant obtained from the primary extract which is obtained by extracting glutathione-containing yeast cells with water at 60 to 90 ° C, and a supernatant of a secondary extract which is obtained by treating the cells recovered from the primary extract with proteolytic enzymes and extracting them with water at a temperature of 60 to 90 ° C.

The proteolytic enzyme is not particularly limited as long as it has an activity to cleave a peptide bond, but an endo-protease, an exo-protease, a mixed enzyme thereof, or an enzyme with complex activity having both activities may be used, or preferably may be an endo-protease or a mixed enzyme of the endo-protease and the exo-protease. The exo -protease includes an amino-terminal hydrolase that acts on the amino terminal and a carboxy-terminal hydrolase that acts on the carboxy-terminus. The endo-protease is serine-protease, cysteine-protease, asparagine-protease, and metallo-proteases depending on amino acid type at an active site.

The proteolytic enzyme of the present invention refers to an enzyme that hydrolyzes proteins of food materials. Preferably, the proteolytic enzyme may be selected from the group consisting of MULTIFECT, Alcalase, Protamex, Neutrase, Esperase, Flavourzyme and Fungal protease. Alcalase is a serine type endoprotease and MULTIFECT is a metallo-protease. The enzyme with complex activity having both endo-peptidase and exo-peptidase in the present invention refers to a peptidase that exhibits endo-peptidase and exo-peptidase activities together, and preferably may be selected from the group consisting of Alcalase, Flavourzyme, and Fungal protease, and more preferably Alcalase or Flavourzyme.

In the present invention, for additional extraction of useful components in the strain, additional extraction is performed by treatment with a proteolytic enzyme after solvent extraction of the cells using water is performed, thereby increasing the dry matter yield of the yeast extract. The dry matter yield of the yeast extract mainly includes proteins and peptides as water-soluble components eluted with water as an extraction solvent. Since the yeast extract in the present specification includes a solvent extract, it includes water-soluble substances eluted from yeast cells and dissolved in water, such as proteins and peptides. Therefore, the yeast extract according to the present invention may contain the protein and peptide content of 40 wt% or more, 50 wt% or more, 60 wt% or more based on 100% of the yeast extract on a solid basis, and may be the protein and peptide content of 95 wt% or less, 90 wt% or less, 85 wt% or less, 80 wt% or less, 75 wt% or less, or 70 wt% or less as an upper limit.

In the enzyme treatment step, a suitable amount of enzyme may be 0.5 to 5ul/ml, but is not limited thereto.

In the method for producing yeast extract according to the present invention, the dry matter yield of the yeast extract may be 20% to 65 wt%, or specifically 30 to 45 wt%.

In the method for preparing the yeast extract or the solvent extract according to the present invention, the step of extracting with water is 3 minutes to 240 minutes, 3 minutes to 220 minutes, 3 minutes to 210 minutes, 3 minutes to 200 minutes, 5 minutes to 240 minutes, 5 minutes to 220 minutes, 5 minutes to 210 minutes, 5 minutes to 200 minutes, 7 minutes to 240 minutes, 7 minutes to 220 minutes, 7 minutes to 210 minutes, 7 minutes to 200 minutes, 9 minutes to 240 minutes, 9 minutes to 220 minutes, 9 minutes to 210 minutes, 9 minutes to 200 minutes, 9 minutes to 190 minutes, 9 minutes to 180 minutes, 9 minutes to 170 minutes, 9 minutes to 160 minutes, 9 minutes to 150 minutes, 9 minutes to 140 minutes, 9 minutes to 130 minutes, or 9 minutes to 125 minutes, or for example, 10 minutes to 120 minutes.

In the manufacturing method for yeast extract or solvent extract according to the present invention, since the yeast extract contains a large amount of proteins and peptides, the maximum extraction yield can be secured achieved by adjusting the extraction time and extraction temperature preferably to a short time in the case of the extraction at a high temperature, or a long time in the case of extraction at a relatively low temperature. For example, when extracting using water at 80 to 90 ° C., it can be set to 60 minutes or less, which is a relatively short time, and for example, 3 to 60 minutes, or 5 to 30 minutes. Alternatively, 7 to 240 minutes, 9 to 240 minutes, or 10 minutes when extraction is performed at a temperature condition of 85 °C or less, for example, 60 to 85 ° C, 60 to 80 °C, or 60 to 75 °C, it can be performed by appropriately selecting the conditions.

In the method for preparing the yeast extract or the solvent extract according to the present invention, the extraction time may be appropriately adjusted according to the amount of the extract solution. For example, it can be adjusted to about 25 minutes when the amount of the extract solution is 10 to 1000 ml, and about 35 minutes when the amount of the extract is 1000 to 2000 ml, but not intended to be limited thereto.

In the method for preparing the yeast extract or the solvent extract according to the present invention, the extraction may be performed while stirring or mixing the extract solution. For example, the extraction can be performed while stirring at 100 to 1000 rpm using a magnetic stirrer, so as to mix the solution well.

The present invention relates to glutathione extraction using yeast containing glutathione and to glutathione extract prepared therefrom, using a hot water extraction method to obtain 20 wt% or more, 22 wt% or more, or 25 wt% or more, for example, 20 wt% to 45wt% of production yield. Furthermore, by performing enzymatic treatment together with hot water extraction, it is possible to obtain extracts in high yields, that is yeast extracts having a high protein content of 65% or more. The extract can be used in various fields of application, such as food and health functional products, etc.

**[EFFECT OF THE INVENTION]**

The present invention relates to glutathione extraction using yeast containing glutathione and glutathione extract prepared therefrom, where the extract is prepared in high yield using a hot water extraction method. Furthermore, it is possible to obtain an extract with a dry matter yield of 60% or more by performing enzymatic treatment together with hot water extraction. Th extract can be used in various applications such as food and health functional products.

**[BRIEF DESCRIPTION OF THE DRAWINGS]**

Fig. 1 shows a tripeptide (glutathione) content pattern over the time passage during extraction using hot water of 90 °C according to an example of the present invention.

Fig. 3 shows a tripeptide (glutathione) content pattern over the time passage during extraction using hot water of 80 °C according to an example of the present invention.

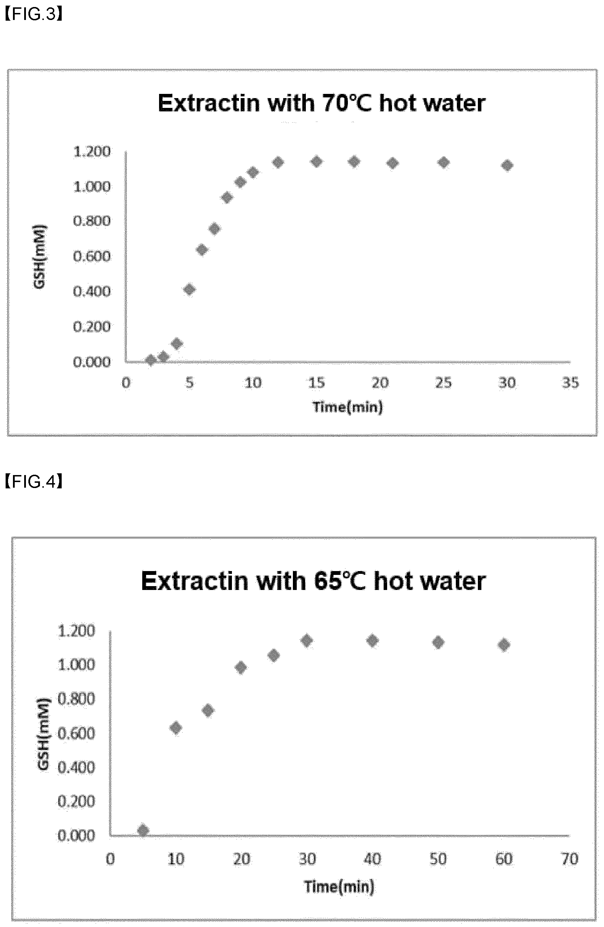

Fig. 3 shows a tripeptide (glutathione) content pattern over the time passage during extraction using hot water of 70 °C according to an example of the present invention.

Fig. 4 shows a tripeptide (glutathione) content pattern over the time passage during extraction using hot water of 65 °C according to an example of the present invention.

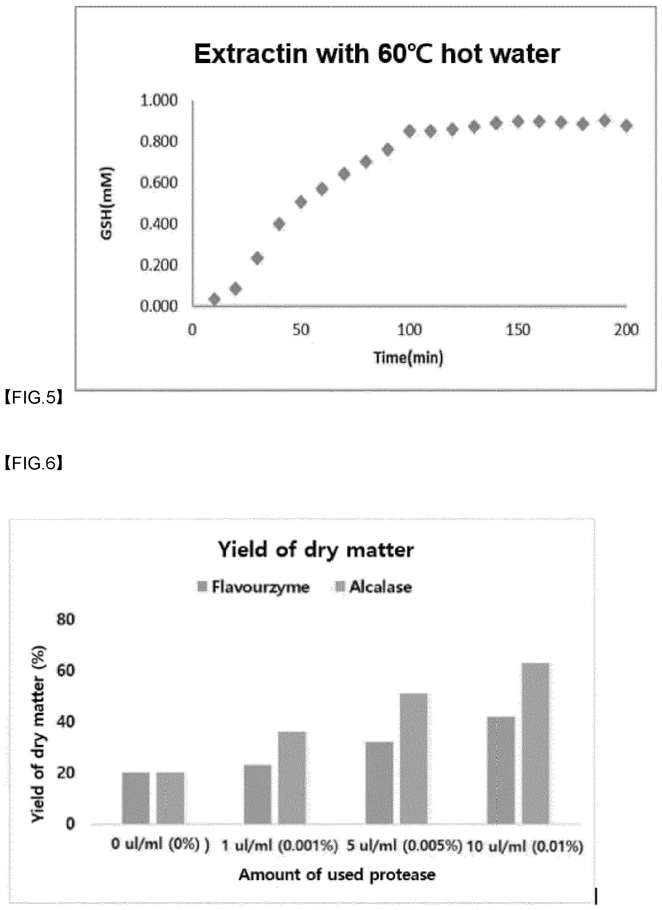

Fig. 5 shows a tripeptide (glutathione) content pattern over the time passage during extraction using hot water of 60 °C according to an example of the present invention.

Fig. 6 is a graph showing the yield (w / w%) of the dried extract obtained content according to the type and concentration of the protease in an example of the present invention.

## [DETAILED DESCRIPTION OF THE EMBODIMENTS]

The present invention will be explained in more detail with reference to the following examples, but the present invention is not intended to be limited to the following examples.

### Example 1: Preparation of glutathione-producing microorganisms

#### 1-1 Microorganism preparation

To screen microorganisms producing glutathione, rice wine (Makgeolli) koji, and traditional sources were purchased at traditional markets across the country and were used them as samples. 1 g of the sample was suspended in 10 mL of 0.85% NaCl, and 100 $\mu$l of the suspension was plated on a YPD (Yeast extract 10 g/L, Peptone 20 g/L, Dextrose 20 g/L) agar plate and the solid culture was performed at 30 °C for 2 days. Among the colonies grown on the solid medium, 150 colonies were isolated by selecting those with different shapes and sizes, and then cultured at 30 °C for 24 hours in a test tube containing YPD broth (yeast extract 10 g/L, Peptone 20g/L, Dextrose 20g/L) with agitation, to obtain the cell culture.

The cell concentration was determined by measuring the absorbance at 600 nm for the cell culture, and the measured absorbance (cell optical density, OD) was 17.8.

#### 1-2: Glutathione content analysis

The cell culture solution was centrifuged to remove the supernatant, and the microbial cells were collected by washing with distilled water once. The collected cells were added with 40-70% ethanol, and the intracellular glutathione was extracted using a fine mixer for 10-30 minutes. After centrifugation of the extraction solution, the supernatant was taken and reacted with 10 mM DTNB (5,5'-Dithiobis-(2-Nitrobenzoic Acid) dissolved in 0.5 M potassium phosphate at pH8.0 buffer for 20 min at 40°C, and the glutathione content (GSH mg/L) was determined by measuring the absorbance at 412 nm. DTNB, commonly used for glutathione content analysis, is known as Ellman's reagent designed to detect thiol compounds. The reaction of GSH with DTNB produces y2-nitro-5-benzoic acid and GSSG, and the concentration of GSH can be calculated by measuring the OD value at 412 nm. The measured glutathione content was 112 mg/L.

#### 1-3: Measurement of glutathione content per cell dry weight (g)

The cell culture solution was centrifuged to remove the supernatant, and the microbial cells were collected by washing with distilled water once. The absorbance was measured for the collected cells, and the dried cell weight was calculated according to the absorbance. Specifically, in order to measure the cell dry weight(g), the cell culture solution was centrifuged to remove the supernatant, and the microbial cells were collected only. The collected cells were washed with 0.9% NaCl and diluted with distilled water to prepare samples with 0.1 to 1 of absorbance.

The absorbance was measured at 600 nm for the diluted cell samples, and the dry cell weight was calculated according to the absorbance. After measuring the absorbance, the cells were filtered under reduced pressure with a 0.2 $\mu$m filter paper. The filter paper with cells remaining was dried at 60 ° C for more than 12 hours, left in a desiccator containing silica gel for more than 6 hours, and then the cell weight was measured. The amount of dried cells was determined by calculating the weight difference between the empty filter paper and the filter paper with the cells filtered out. Therefore, it was possible to determine the dry cell concentration (g/L) according to the absorbance value.

After measuring the absorbance, glutathione was extracted from the cells in substantially the same manner as in Examples 1-2, and the extraction solution was centrifuged to take the supernatant, and then the produced amount of glutathione (g/L) was measured. The measured amount of glutathione was divided by the calculated dry cell weight (g/L) and then multiplied by 100 to calculate GSH% per dry cell weight (g). The glutathione content per dry cell weight (g) for the statin (productivity, GSH(%)/g-cell) was 1.6%.

1-4: Alcohol dehydrogenase (ADH) activity assay

The ADH activity was analyzed using an ADH Activity Assay Kit (Abcam). After culturing for 24 to 48 hours at 30 °C in YPD medium, the cells were collected to $1 \times 10^6$ CFU / ml. The collected cells were washed with distilled water, and then were added with ADH assay buffer, to disrupt the cell walls using a bead beater. As the composition of the reaction solution, 50 µl of sample or NADH standard material was mixed with 82 µl of ADH assay buffer, 8 µl of Developer, and 10 µl of Isopropanol for each concentration. After reacting at 37 °C. for 3 minutes, the absorbance of the experimental group (A0) and the control group was measured at 450 nm. After an additional reaction at 37 °C. for 30 minutes, the change in absorbance at 450 nm was measured, and the amount of NADH produced per unit time (minute) was calculated.
Therefore, the strain is a SYC-PR20 strain having a high OD value and high glutathione production, which is *Candida utilis* SYC-PR20 having accession number of KCCM 12777P. The strain has excellent properties such as a glutathione yield of 1.6% and an ADH activity of 0.26 mU/ml, and has the 18S rDNA sequence of SEQ ID NO: 1.

1-5: Acquisition of Microbial cells

As a seed culture, 3mL of the cell culture was weighed and inoculated into an flask containing 50mL of YPD medium. Shaking culture was performed at 30° C for 48 hours, and 1 ml of the culture solution was sampled and centrifuged at 12,000 rpm for 5 minutes. The medium components of the supernatant were removed, the cells were washed with the same amount of distilled water, and cells were obtained by centrifugation at 12,000 rpm for 5 minutes.

**Example 2: Extraction of glutathione depending extraction temperature**

2-1: Extraction sample

As a method for recovering tripeptide (glutathione) in the cells, the cells were suspended with addition of distilled water to adjust the cell concentration to a certain concentration, and extraction was performed under various extraction temperature to determine the optimal extraction conditions for tripeptide (glutathione).
The glutathione-producing strain, *Candida utilis* SYC-PR20 obtained in Example 1 was used, and the cells were obtained in the same manner as in Example 1 and suspended in distilled water to adjust the OD600 value to 100 to prepare a diluted cell solution. The diluted cell solution was used as a sample for hot water extraction.

2-2: Glutathione Extraction

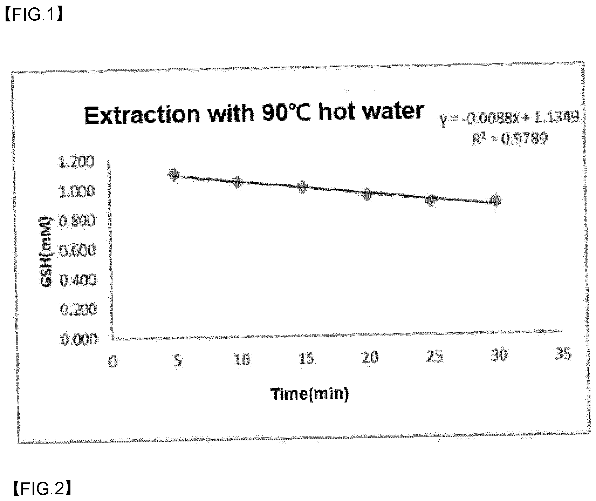

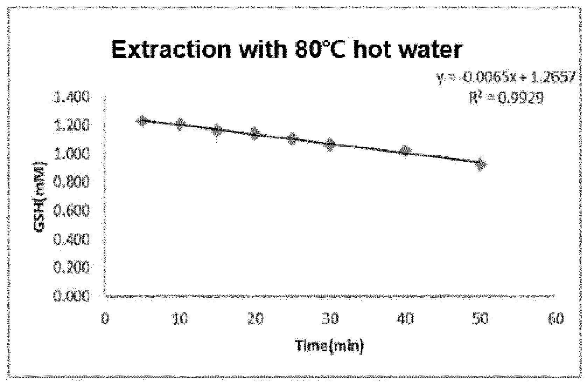

The extraction sample was the prepared diluted cell solution that the cells were suspended in distilled water to adjust the OD600 value to 100. The glutathione extraction for the samples were carried out by using five waters of 90°C, 80°C, 70°C, 65°C, or 60°C.
Specifically, the extraction temperature was adjusted using a constant temperature water bath, and the extraction was performed by adjusting the amount of the extraction liquid to 100 mL. In the extraction process, samples to be analyzed were taken at intervals of 5 or 10 minutes and the content of glutathione contained in the extract was quantified by the DTNB color development method.
After centrifuging the extraction solution, the supernatant was taken and reacted at 40°C for 20 minutes with 10mM DTNB (5,5'-Dithiobis-(2-Nitrobenzoic Acid)) dissolved in 0.5M potassium phosphate pH8.0 buffer, and absorbance at 412nm was measured to determine the glutathione content, and the glutathione content (GSH mg/L). DTNB is mainly used for glutathione analysis and is known as Ellman's reagent designed to detect thiol compounds. The reaction between DTNB and GSH produces 2-nitro-5-benzoic acid in yellow color and GSSG, and the concentration of GSH can be calculated by measuring the OD value at 412 nm. GSSG is reduced to GSH by glutathione reductase and forms a recycling system that reacts with DTNB again. The analysis results of the glutathione content contained in the extract, depending on the temperature of hot water are shown in FIGs.1 to 5, respectively. The maximum content of extracted glutathione and the time to reach the maximum content depending on the temperature of hot water shown in FIGs. 1 to 5 are represented in Table 1 below.

[Table 1]

| Extraction temperature(°C) | Maximum content of GSH (mM) | Extraction time to reach the maximum content of GSH (minute) |
|---|---|---|
| 90 | 1.10 | 5 |
| 80 | 1.23 | 5 |
| 70 | 1.12 | 10 |
| 65 | 1.14 | 40 |
| 60 | 0.90 | 190 |

According to the extraction results in Table 1 and FIGs.1 to 5, the extraction time to reach the maximum GSH content at extraction temperatures of 80 ° C and 90 ° C was about 5 minutes, and the glutathione content slightly decreased as the extraction time passed. Therefore, it was preferable to extract glutathione at high temperature and for a short time. In addition, as the extraction temperature decreased, the extraction time to reach the maximum GSH content tended to increase, and the maximum glutathione extraction content gradually decreased at temperatures of 60 °C or lower.

Therefore, considering the maximum content of glutathione and the time to reach the maximum content of glutathione, and the cost of elevating temperature when applied industrially and the feasibility of controlling temperature when extracting at high temperature for a short time, it is recommended that the extraction temperature is 70° C. Under the condition that the extraction temperature was 70°C, the maximum extraction content of glutathione was shown when the extraction time was 10 minutes or more, and was almost constant until 20 minutes.

**Example 3: Evaluation of the dry matter yield depending on the cell concentration**

In this Example, optimal extraction conditions were set by measuring the dry matter content, dry matter yield, and GSH content of the yeast extract depending on the cell concentration of the sample used for extraction.

Specifically, in the process of preparing the extraction sample prepared in Example 2-1, the cells were suspended in distilled water to adjust the OD600 value to 100 to 500, to prepare six diluted cell solutions having different cell concentrations (Table 2).

Using the prepared diluted cell solutions, glutathione was extracted in substantially the same way as in Example 2-2 using hot water at 70 ° C, and PASTE was removed from the resulting extracted solution to obtain a crude extract solution, and the volume of the crude extract solution was measured and shown in table 2. The crude extract solution was freeze-dried to prepare a dry powder of the crude extract. 10 ml of the crude extract was freeze-dried in a speed vacuum concentrator, and the weight of the obtained dry powder was measured, and the dry weight (g/L) was shown in Table 2 below. Table 2 shows the total dry weight (g) of the dry matter obtained from the crude extract by multiplying the dry matter weight by the recovered liquid amount. The dry matter yield was obtained by inputting the obtained total dried weight (g) into Equation 1 below, and expressed as w/w% in Table 2 below. In Equation 1 below, OD is the optical density measured at a wavelength of 600 nm and means the cell concentration.

[Formula 1]

$$\text{Yield of dry matter} = \text{Total weight of dry matter (g)} / (OD * 0.4)*100$$

[Table 2]

| Cell OD | Weight of dry matter | Recovered liquid amount | Total weight of dry matter | yield of dry matter | GSH content |
|---|---|---|---|---|---|
| | g/L | L | 9 | %(w/w) | %(w/w) |
| 100 | 13.96 | 0.91 | 12.73 | 31.83 | 5.85 |
| 200 | 29.16 | 0.85 | 24.73 | 30.91 | 6.53 |
| 250 | 39.32 | 0.81 | 32.01 | 32.01 | 6.23 |

(continued)

| Cell OD | Weight of dry matter | Recovered liquid amount | Total weight of dry matter | yield of dry matter | GSH content |
|---------|----------------------|-------------------------|----------------------------|---------------------|-------------|
| | g/L | L | 9 | %(w/w) | %(w/w) |
| 300 | 48.80 | 0.77 | 37.38 | 31.15 | 6.26 |
| 400 | 69.87 | 0.70 | 48.77 | 30.48 | 6.13 |
| 500 | 75.95 | 0.63 | 47.85 | 23.92 | 6.93 |

As shown in Table 2, It was confirmed that the dry matter yield of the yeast extract was 23.92% (w/w) for 500 of OD600, and the dry matter yield of the yeast extract was the highest for 250 of the cell concentration OD600 value is 250. Therefore, since the total dry matter content and GSH content% increase as the cell concentration increases, the higher the cell concentration (OD) value is, the more advantageous it is. However, as the cell OD value increases, the amount of recovered liquid decreases and the dry matter yield decreases. Therefore, it is preferable to adjust the cell concentration appropriately by considering the yield of dry product. Accordingly, in consideration of the dry matter yield and GSH content as a result of the above experiments, extraction may be performed at an OD value of 50 to 500, or preferably 100 to 400.

**Example 4: Glutathione extraction using protease**

In this Example, it was attempted to increase the extraction content of glutathione and increase the dry matter yield of yeast extract by treating with proteolytic enzyme (protease). Therefore, in the step before performing the hot water extraction, the diluted cell solution was treated with enzyme, and the GSH content extracted and eluted was determined as a measuring index in order to directly test the effect of the enzyme treatment. The use of protease accelerates cell degradation to quickly elute intracellular glutathione, and the eluted glutathione is degraded over time, resulting in a low content. Therefore, an enzyme with a large decrease in glutathione content depending on the time passage and increased amount of enzyme, should be determined in this test.

As proteolytic enzymes, 4 types in total including 2 types of Novozyme's Alcalase (trade name) and Flavourzyme (trade name), and 2 types of Multifect (trade name) and papain were selected and tested. The general characteristics of proteases act as two types of endo-type (alcalase) and exo-type (papain), Flavourzyme is a mixture of the two types.

Specifically, samples for enzyme treatment and extraction were prepared in the same manner as in Example 2-1. The prepared cell dilution sample was dispensed into a 1.5 ml E-Tube in a reaction volume of 1 ml, and the four enzymes were added to the dispensed cell dilution solution in an amount of 0, 1, 2, 5, 10, and 15 $\mu$l / ml, respectively. The enzyme reaction was performed at a temperature of 50 ° C for 120 minutes, with taking samples to be analyzed at 30 minutes, 60 minutes and 120 minutes, and was analyzed using the DTNB color development method in substantially the same manner as in Example 2-2. Table 3 shows the GSH content (uM) depending on the type and the used amount used (ul/ml) of the four enzymes.

[Table 3]

| Enzyme | minute | 0ul/ml | 1ul/ml | 2ul/ml | 6ul/ml | 10ul/ml | 15ul/ml |
|--------|--------|--------|--------|--------|--------|---------|---------|
| Alcalase | 30 | 0.87 | 0.67 | 0.60 | 0.45 | 0.34 | 0.27 |
| Alcalase | 60 | 0.65 | 0.38 | 0.30 | 0.17 | 0.10 | 0.07 |
| Alcalase | 120 | 0.31 | 0.03 | 0.03 | 0.02 | 0.01 | 0.01 |
| Flavourzyme | 30 | 0.80 | 0.76 | 0.71 | 0.60 | 0.45 | 0.33 |
| Flavourzyme | 60 | 0.65 | 0.57 | 0.52 | 0.40 | 0.25 | 0.16 |
| Flavourzyme | 120 | 0.28 | 0.19 | 0.15 | 0.08 | 0.05 | 0.04 |
| Multifec | 30 | 0.83 | 0.82 | 0.81 | 0.81 | 0.77 | 0.71 |
| Multifec | 60 | 0.64 | 0.63 | 0.62 | 0.62 | 0.57 | 0.49 |
| Multifec | 120 | 0.28 | 0.24 | 0.25 | 0.24 | 0.19 | 0.12 |
| papain | 30 | 0.83 | 0.82 | 0.86 | 1.31 | 1.99 | 2.56 |

(continued)

| Enzyme | minute | 0ul/ml | 1ul/ml | 2ul/ml | 6ul/ml | 10ul/ml | 15ul/ml |
|---|---|---|---|---|---|---|---|
| papain | 60 | 0.63 | 0.51 | 0.53 | 0.97 | 1.64 | 2.22 |
| papain | 120 | 0.29 | 0.10 | 0.10 | 0.35 | 0.91 | 1.46 |

When glutathione-containing cells were treated with 4 types of proteases, the change in GSH content depending on the change in protease concentration was investigated. In the case of Alcalase and Flavourzyme, the change in the GSH content was detected depending on the enzyme concentration, which indirectly confirmed their action on the cell wall. On the other hand, there was no change at enzyme concentration for multifec. Papain affected the GSH coloring method, and thus was excluded from the experiment. The two enzymes (Alcalase and Flavourzyme) with significant changes in GSH content were selected.

**Example 5: Glutathione extraction using protease and hot water extraction**

After performing solvent extraction of the cells using hot water, additional extraction was performed by treatment with a proteolytic enzyme for additional extraction of useful components in the cells, in order to increase the dry matter yield of yeast extract. The dry matter yield of the yeast extract mainly includes proteins and peptides, which are as water-soluble components eluted with water as an extraction solvent.

Specifically, samples for enzyme treatment and extraction were prepared by suspending cells in distilled water in the same manner as in Example 2-1 to adjust the OD600 value to 100 to prepare 100 mL of cell dilution samples. The prepared sample was dispensed into three 30 to 35ml flacon tubes, respectively. In substantially the same manner as in Example 2, glutathione was extracted for 25 minutes using hot water from the cell dilution solution dispensed into the flacon tube at a temperature of 70 °C. The extract was centrifuged and the supernatant was recovered to prepare a crude extract.

After adding 90mL of distilled water to the cell paste remaining after recovering the supernatant, two kinds of Novozyme's Alcalase and Flavorzyme were added at an enzyme concentration of 1 ul/ml (0.001%), 5 ul/ml (0.005%), and 10 ul/ml (0.01%), respectively, and an enzyme reaction was performed at a temperature of 50° C for 120 minutes.

The enzyme reaction product solution was secondarily extracted for glutathione for 25 minutes using hot water at a temperature of 70 °C. The secondary extract was centrifuged and the supernatant was recovered to prepare a secondary crude extract.

The glutathione content contained in the extract sample obtained by mixing the first crude extract and the second crude extract was analyzed by the DTNB color development method in substantially the same manner as in Example 2-2.

In addition, the mixed sample was lyophilized to prepare a dry powder of the crude extract, and the weight of the dry powder obtained after freeze-drying 10 ml of the crude extract in speed vacuum concentrator was measured to obtain a dry weight (g/L). The total weight (g) of the dry matter of the crude extract was calculated by multiplying the dry weight with the recovered liquid amount. The total dry matter weight (g) obtained above was input into Equation 1 to obtain the dry matter yield (w / w%), which is shown in Table 4 as w/w%. In addition, the dry matter yield of the extract depending on the protease type and concentration is shown in FIG. 6.

[Table 4]

| Enzyme | 0 ul/ml of used enzyme | 1 ul/ml of used enzyme | 5 ul/ml of used enzyme | 10 ul/ml of used enzyme |
|---|---|---|---|---|
| Flavourzyme Treatment | 20%(w/w) | 23%(w/w) | 32%(w/w) | 42%(w/w) |
| Alcalase Treatment | 20%(w/w) | 36%(w/w) | 51%(w/w) | 63%(w/w) |

As shown in Table 4, when the secondary extract was obtained by hot water extraction after treatment with a proteolytic enzyme, the dried yeast extract could be obtained in high yield advantageously without reducing the content of glutathione, and Alcalase was more preferable as a proteolytic enzyme. Within the experimental ranges, the dry matter yield of yeast extract increased as the amount of treated enzyme increased.

**Claims**

1. A method of producing an yeast extract containing 0.5 to 20 % by weight of glutathione based on solid content, comprising a step of obtaining a solvent extract by extracting yeast cells containing glutathione or suspension of the cells, with water at 60 to 90 °C.

2. The method of claim 1, wherein the step of obtaining a solvent extract comprises obtaining a primary extract obtained by extracting yeast cells containing glutathione with a water at 60 to 90 °C, and a secondary extract obtained by treating the cells recovered from the primary extract with a proteolytic enzyme and extracting with water.

3. The method according to claim 1, further comprising a step of obtaining a dried product by drying the solvent extract.

4. The method of claim 3, wherein the dry matter yield of the yeast extract is 20 to 65 % by weight.

5. The method according to claim 3, wherein a time for extracting with water in the step of obtaining the solvent extract is 10 minutes to 120 minutes.

6. The method according to claim 1, wherein the cell concentration of the suspension of the cells has 50 to 500 of an optical density (OD) value measured at 600 nm.

7. The method of claim 1, wherein the yeast is *Candida utilis.*

8. The method of claim 2, wherein the proteolytic enzyme is endo-protease or mixed enzymes of endo-protease and exo-protease.

9. The method of claim 2, wherein the treated amount of the proteolytic enzyme is 0.5 to 5ul/ml.

10. A yeast extract comprising a solvent extract using water at a temperature of 60 to 90 ° C for yeast cells containing glutathione or suspension of the cells, and containing 0.5 to 20 % by weight of glutathione based on solid content.

11. The yeast extract according to claim 10, wherein the solvent extract is obtained by extracting proteolytic enzyme-treated yeast cells or a suspension of the cells with water.

12. The yeast extract of claim 11, wherein the solvent extract comprises a primary extract obtained by extracting yeast cells containing glutathione or suspension of the cell with water at 60 to 90 °C, and a secondary extract obtained by treating the cells recovered from the primary extract with a proteolytic enzyme and extracting with water.

13. The yeast extract of claim 11, wherein the proteolytic enzyme is endo-protease or mixed enzymes of endo-protease and exo-protease.

14. The yeast extract of claim 11, wherein the treated amount of the proteolytic enzyme is 0.5 to 5ul/ml.

15. The yeast extract of claim 10, wherein the yeast is *Candida utilis.*

16. The yeast extract according to claim 10, wherein the yeast extract is a dried product obtained by drying the solvent extract.

【FIG.1】

Extraction with 90℃ hot water

$y = -0.0088x + 1.1349$
$R^2 = 0.9789$

GSH(mM)

Time(min)

【FIG.2】

【FIG.3】

Extractin with 70℃ hot water

【FIG.4】

Extractin with 65℃ hot water

【FIG.5】

**Extractin with 60°C hot water**

【FIG.6】

**Yield of dry matter**

## INTERNATIONAL SEARCH REPORT

International application No.

**PCT/KR2021/019679**

| A. | CLASSIFICATION OF SUBJECT MATTER |
|---|---|

**C12N 1/16**(2006.01)i; **C12P 21/02**(2006.01)i

According to International Patent Classification (IPC) or to both national classification and IPC

| B. | FIELDS SEARCHED |
|---|---|

Minimum documentation searched (classification system followed by classification symbols)

C12N 1/16(2006.01); A23L 1/28(2006.01); A23L 27/10(2016.01); A23L 33/14(2016.01); A23L 33/145(2016.01); A61K 31/52(2006.01); A61K 36/064(2006.01); C12P 21/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 글루타치온(glutathione), 칸디다 유틸리스(Candida utilis), 열수 추출(hot water extraction), 단백질 분해효소(protease)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2018-0012755 A (KOHJIN LIFE SCIENCES CO., LTD.) 06 February 2018 (2018-02-06)<br>See abstract; claims 1-5; and paragraphs [0014]-[0017]. | 1-16 |
| X | US 2019-0307823 A1 (KOHJIN LIFE SCIENCES CO., LTD.) 10 October 2019 (2019-10-10)<br>See abstract; claims 1-3; and paragraphs [0017]-[0020]. | 1-16 |
| X | US 2018-0070623 A1 (AJINOMOTO CO., INC.) 15 March 2018 (2018-03-15)<br>See abstract; claims 1-18; and paragraphs [0040]-[0041]. | 1,3-7,10,15-16 |
| Y | | 2,8-9,11-14 |
| X | JP 2004-283125 A (KOHJIN CO., LTD.) 14 October 2004 (2004-10-14)<br>See abstract; claims 1-5; and paragraphs [0014]-[0015]. | 1,3-7,10,15-16 |
| Y | | 2,8-9,11-14 |

☑ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 March 2022** | **31 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/019679** |

### C.     DOCUMENTS CONSIDERED TO BE RELEVANT

| Category* | Citation of document, with indication, where appropriate, of the relevant passages | Relevant to claim No. |
| --- | --- | --- |
| Y | KR 10-2011-0118492 A (SEMPIO FOODS COMPANY) 31 October 2011 (2011-10-31)<br>See claims 7-12; and paragraphs [0036]-[0039]. | 2,8-9,11-14 |

Form PCT/ISA/210 (second sheet) (July 2019)

**INTERNATIONAL SEARCH REPORT**
**Information on patent family members**

International application No.

**PCT/KR2021/019679**

| Patent document cited in search report | | | Publication date (day/month/year) | Patent family member(s) | | | Publication date (day/month/year) |
|---|---|---|---|---|---|---|---|
| KR | 10-2018-0012755 | A | 06 February 2018 | CN | 107613996 | A | 19 January 2018 |
| | | | | JP | 6789214 | B2 | 25 November 2020 |
| | | | | TW | 201701890 | A | 16 January 2017 |
| | | | | WO | 2016-194778 | A1 | 08 December 2016 |
| US | 2019-0307823 | A1 | 10 October 2019 | CN | 109310725 | A | 05 February 2019 |
| | | | | EP | 3459554 | A1 | 27 March 2019 |
| | | | | EP | 3459554 | B1 | 27 October 2021 |
| | | | | TW | 201740965 | A | 01 December 2017 |
| | | | | US | 2020-0246403 | A1 | 06 August 2020 |
| | | | | WO | 2017-199951 | A1 | 23 November 2017 |
| US | 2018-0070623 | A1 | 15 March 2018 | EP | 3295809 | A1 | 21 March 2018 |
| | | | | TW | 201705870 | A | 16 February 2017 |
| | | | | WO | 2016-186010 | A1 | 24 November 2016 |
| JP | 2004-283125 | A | 14 October 2004 | None | | | |
| KR | 10-2011-0118492 | A | 31 October 2011 | KR | 10-1150148 | B1 | 08 June 2012 |

Form PCT/ISA/210 (patent family annex) (July 2019)